# FASCICULE DE BREVET EUROPEEN

(11) **EP 0 704 000 B1**
(45) Date de publication et mention de la délivrance du brevet: **02.04.1997**
(21) Numéro de dépôt: 94917047.6
(22) Date de dépôt: 24.05.1994
(51) Int. Cl.: D04H 1/48, D04H 13/00, A61F 13/46

(54) **MATERIAU NON-TISSE COMPOSITE, PROCEDE DE FABRICATION ET SON APPLICATION A TOUT ARTICLE D'HYGIENE ABSORBANT**
VERBUNDVLIESSTOFF, HERSTELLUNGSVERFAHREN UND SEINE ANWENDUNG FÜR ALLE SAUGFÄHIGEN HYGIENISCHEN ARTIKEL
COMPOSITE NON-WOVEN MATERIAL, METHOD OF MANUFACTURE AND APPLICATION TO ABSORBENT SANITARY ARTICLES

(30) Priorité: 26.05.1993 FR 9306327
(43) Date de publication de la demande: 03.04.1996
(73) Titulaire: PEAUDOUCE, 59126 Linselles (FR)
(72) Inventeur: KOCZAB, Jean-Pierre, F-59910 Bondues (FR)
(74) Mandataire: Casalonga, Axel
(86) Numéro de dépôt international: FR9400607
(87) Numéro de publication internationale: WO9428222

(56) Documents cités:
- EP-A- 0 252 041
- EP-A- 0 352 208
- DE-A- 3 029 315
- US-A- 3 206 351
- US-A- 3 936 555
- US-A- 3 956 560

## Description

L'invention concerne, d'une manière générale, un nouveau matériau non-tissé, qui lorsqu'il est utilisé comme voile ou feuille de surface ou en complément du voile de surface comme bande dans la zone d'entre-jambes dans un article d'hygiène absorbant, tel qu'une couche-culotte ou garniture pour incontinents, permet une meilleure isolation de la peau de l'utilisateur de la partie absorbante de l'article d'hygiène. En particulier, ce nouveau matériau favorise le temps de transpercement par les fluides corporels, la résistance au remouillage, et ne peluche pas.

D'une manière générale, les articles d'hygiène absorbants, tels que des couche-culottes et garnitures pour incontinents, comprennent une couche externe en matériau imperméable aux liquides, un coussin en matériau absorbant et un voile ou feuille de surface perméable aux fluides corporels, tels que l'urine, de dimension et de forme analogues à celles de la couche externe imperméable de l'article. Ce voile de surface perméable aux fluides corporels a pour but d'isoler la peau du coussin absorbant humidifié. Par conséquent, le voile de surface doit avoir un degré de douceur convenable et assurer une isolation voulue entre la peau et le coussin absorbant. Le coussin absorbant a pour fonction d'absorber les fluides et par conséquent doit présenter une vitesse d'absorption importante ainsi qu'une grande capacité d'absorption. Un coussin absorbant particulièrement efficace est décrit dans le document EP-A-0 232729. Ce coussin ou matelas absorbant se compose d'une nappe de fibres absorbantes longues doublée sur ses faces d'une couche de ouate de cellulose. La nappe doublée par les couches de ouate est aiguilletée depuis les deux faces.

Dans les articles d'hygiène absorbants de tels matelas ou coussins absorbants sont recouverts par un voile de surface ou par une bande dans la zone d'entre-jambes, généralement en matériau non-tissé, qui a pour but d'isoler la peau du coussin absorbant et qui doit assurer un contact agréable avec la peau et l'isolation voulue avec le coussin absorbant. Ces voiles ou feuilles de surface et bandes de zone d'entre-jambes doivent présenter comme propriétés essentielles un contact agréable avec la peau, une vitesse de traversée par les fluides corporels importante, une bonne résistance au remouillage et ne pas pelucher.

Le document FR-A-2 588 285 décrit un textile non-tissé multi-couches ayant au moins deux couches de voile non-tissé, l'une des couches étant formée de fibres de section transversale bilobée et l'autre couche étant formée de fibres de section transversale trilobée. Chaque couche de voile est de préférence obtenue par la technique de liaison au filage (Spun bonded) et les deux couches de voile sont réunies pour former le non-tissé multicouche par liaison thermique en des zones compactées et discontinues.

Le document WO 87/07117 décrit un article d'hygiène absorbant comprenant un corps absorbant entouré d'une enveloppe. Cette enveloppe ou voile de surface est constituée de deux couches en matériau non-tissé. La première couche de matériau non-tissé, en contact avec la peau de l'utilisateur, est constituée d'une mince couche de tissus fibreux lié au filage en un matériau hydrophobique, et la seconde couche en contact avec le corps absorbant est une couche fibreuse hydrophobique de tissu de fibres liée par fusion, de construction similaire à la première couche. Ces deux couches de voile de surface ne sont pas liées entre elles dans la zone destinée à venir en contact avec le corps de l'utilisateur.

Le document WO 88/05269 concerne un voile de surface pour un article absorbant jetable composé d'au moins deux couches de matériau non-tissé qui peuvent être identiques ou différentes et qui sont réunies par des lignes d'adhésif formant un motif ouvert.

On a également récemment mis au point un nouveau matériau non-tissé composite comprenant au moins une première couche constituée par un non-tissé (de préférence lié au filage) et, sur cette première couche une nappe de fibres de type cardé, la nappe de fibres de type cardé étant liée à la couche de base par aiguilletage.

Un autre matériau non-tissé composite récemment mis au point comprend une première couche en un non-tissé (de préférence lié au filage), une nappe de fibres de type cardé et une seconde couche en un non-tissé (de préférence lié au filage) de grammage inférieur à la première couche de non-tissé, la nappe de fibres de type cardé étant disposée entre la première et la deuxième couche de non-tissé. et l'ensemble étant lié par aiguilletage.

Ces matériaux présentent d'excellents temps de traversée par les fluides corporels et une excellente résistance au remouillage. Toutefois, ces matériaux ont une tendance prononcée au peluchage lorsqu'ils sont utilisés comme voile de surface ou bande d'entre-jambes dans des articles d'hygiène absorbants, tels que des couches-culottes.

La présente invention a donc pour but de fournir un matériau non-tissé composite qui, lorsqu'il est utilisé comme voile de surface ou bande d'entre-jambes dans un article d'hygiène absorbant, présente une excellente vitesse de traversée par les fluides corporels, une bonne résistance au remouillage et ne peluche pas.

L'invention a également pour but de fournir un procédé de fabrication d'un tel matériau non-tissé composite.

La présente invention a enfin pour but de fournir un article d'hygiène absorbant comportant un voile de surface ou une bande de zone d'entre-jambes formé d'un tel matériau non-tissé composite.

Selon la présente invention, on réalise un matériau non-tissé composite caractérisé en ce qu'il comprend une nappe de fibres de type cardé perméable aux fluides corporels, comportant au moins une première couche de fibres de type cardé et une seconde couche de fibres de type cardé, les fibres de la première couche ayant un denier supérieur aux fibres de la seconde couche, les deux couches étant réunies entre-elles par aiguilletage.

Dans une réalisation recommandée du matériau non-tissé composite selon l'invention, au moins une des couches de fibres de type cardé comprend des fibres choisies parmi des fibres à bas point de fusion, des fibres bi-composants, et des fibres mélangées avec un liant tel qu'une colle réactivable à chaud ou une poudre à bas point de fusion, et les fibres des couches sont en outre liées entre elles par thermofusion.

Dans une autre réalisation recommandée de l'invention le matériau composite comprend en plus des première et deuxième couches de fibres de type cardé, une troisième couche en un non-tissé classique. perméable aux fluides, par exemple de type lié au filage (spun) ou lié par fusion, de faible grammage, liée à l'une des couches de type cardé. par exemple la couche de fibres de type cardé de plus faible denier.

L'invention fournit également un procédé de fabrication d'un matériau non-tissé composite comprenant les étapes consistant à :
alimenter une carde, dans le sens de la largeur, avec des premières fibres et des secondes fibres, les premières fibres ayant un denier supérieur aux secondes fibres, pour former des bandes adjacentes ou juxtaposées de fibres de type cardé des premières et secondes fibres;
fournir les bandes adjacentes de fibres de type cardé à un dispositif étaleur-nappeur pour croiser les bandes et former une nappe constituée des bandes superposées des premières et secondes fibres;
fournir cette nappe à un dispositif étireur de nappe pour étirer les fibres des bandes et les orienter dans le sens du défilement tout en augmentant le taux d'orientation verticale desdites fibres afin d'obtenir, à la sortie du dispositif étireur de nappe, une nappe de fibres comprenant une première couche des premières fibres et une seconde couche des secondes fibres; et
soumettre la nappe formée à un aiguilletage depuis au moins l'une de ses faces.

Dans une réalisation recommandée du procédé selon l'invention, au moins une des couches de la nappe comprend des fibres choisies parmi les fibres à bas point de fusion, les fibres bi-composants, et les fibres mélangées avec un liant tel qu'une colle réactivable à chaud ou une poudre à bas point de fusion et le procédé comprend en outre la liaison entre elles des fibres des couches par thermofusion, par exemple par réactivation au moyen d'air chaud traversant la nappe.

Le procédé de la présente invention peut, en outre, comporter une étape supplémentaire consistant à lier par exemple par thermofusion, une troisième couche d'un non-tissé classique, de faible grammage, à l'une des couches de fibres de type cardé, par exemple à la couche de fibres de plus faible denier.

Selon la présente invention on réalise également un article d'hygiène absorbant tel qu'une couche-culotte qui comprend une couche externe en matériau imperméable aux liquides corporels, un coussin absorbant perméable aux fluides corporels et sur ce coussin absorbant soit un voile ou feuille de surface, soit un voile de surface et une bande de zone d'entre-jambes, le voile de surface. lorsqu'il est utilisé seul, étant constitué du matériau non-tissé composite selon l'invention et la bande de zone d'entre-jambes étant constituée du matériau non-tissé composite selon l'invention et le voile de surface. d'un non-tissé, de préférence hydrophobe, lorsqu'on utilise conjointement une bande de zone d'entre-jambes et un voile de surface. Dans l'article d'hygiène le voile de surface ou la bande de zone d'entre-jambes constitué par le matériau composite selon l'invention, est disposé de telle sorte que la première couche de fibres de type cardé de plus grand denier soit en contact direct avec la surface interne du coussin absorbant, et par conséquent la deuxième couche de fibres de type cardé de plus faible denier, ou la troisième couche en non-tissé classique soit en contact direct avec la peau de l'utilisateur ou en contact avec la surface externe du voile de surface dans le cas où l'article comporte un voile de surface en non-tissé classique.

Dans une réalisation recommandée l'invention fournit un article d'hygiène absorbant tel qu'une couche-culotte, qui comprend une couche externe en matériau imperméable aux liquides corporels, un coussin absorbant perméable aux fluides corporels fixé à la couche externe, la couche externe et le coussin absorbant comprenant des parties d'extrémité opposées élargies réunies par une zone d'entre-jambes plus étroite, une feuille de surface, de préférence hydrophobe, une bande de zone d'entre-jambes, perméable aux fluides corporels, disposée entre le coussin et la feuille de surface et de largeur analogue à la zone d'entre-jambes du coussin et de longueur au moins égale à celle du coussin, cette bande de zone d'entre-jambes étant constituée du matériau non-tissé composite selon l'invention, la première couche de fibres de type cardé de plus grand denier étant directement en contact avec la surface interne du coussin absorbant, lorsque le matériau composite selon l'invention est uniquement constitué de couches de fibres de type cardé, et de préférence la couche en non-tissé classique constituant la couche la plus interne de la bande de zone d'entre-jambes lorsque le matériau composite selon l'invention comprend en outre une telle couche en non-tissé classique.

Dans une réalisation particulièrement recommandée, la feuille de surface comporte une découpe longitudinale médiane formant une ouverture, de préférence de forme oblongue, et une bande intermédiaire, en non-tissé classique, de dimension analogue à celle de la bande de zone d'entre-jambes est disposée directement sur la bande de zone d'entre-jambes, en dessous de la feuille de surface.

La bande de zone d'entre-jambes est en général liée au coussin absorbant par tout moyen approprié, et en particulier par collage sur le bord du coussin absorbant.

La suite de la description se réfère aux figures annexées qui représentent respectivement :
figure 1, une vue schématique en coupe d'un matériau non-tissé composite selon l'invention;
figure 2, une vue de dessus, en partie arrachée, d'un article d'hygiène absorbant tel qu'une couche-culotte comportant une bande de zone d'entre-jambes constituée du matériau non-tissé composite selon l'invention; et
figure 3, une vue en coupe faite suivant la ligne m-m de la figure 2.

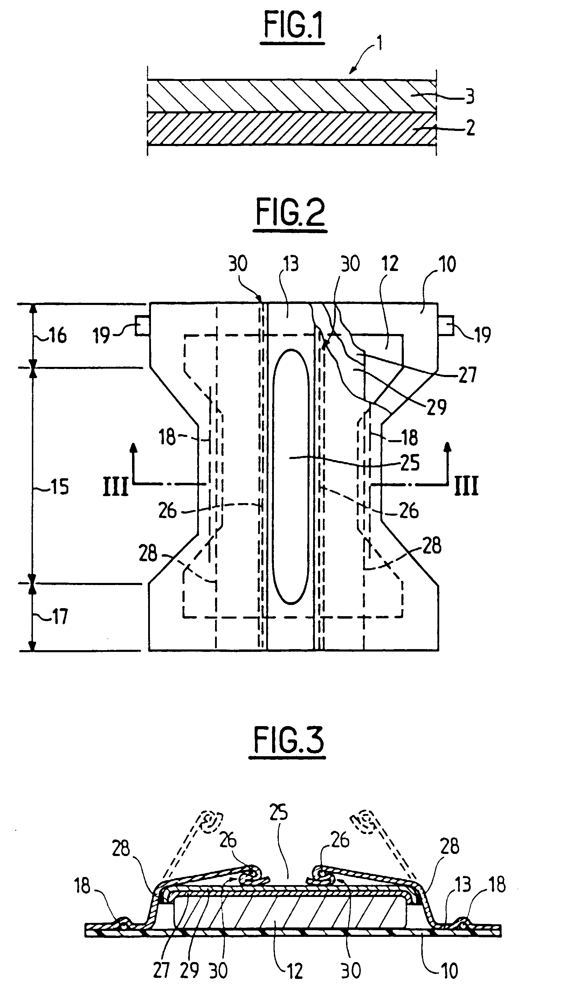

En se référant à la figure 1 on a représenté schématiquement en coupe un matériau non-tissé composite 1 selon l'invention. Ce matériau comprend une première couche 2 constituée de fibres de type cardé et une seconde couche 3 également constituée de fibres de type cardé. Les fibres de la première couche 2 de fibres de type cardé ont un denier supérieur aux fibres de la seconde couche 3 de fibres de type cardé. De préférence, les fibres de la première couche 2 ont un denier compris entre 3 et 8 et les fibres de la seconde couche 3 ont un denier compris entre 1 et 3. Dans une réalisation particulièrement recommandée, les fibres de la première couche 2 de type cardé ont un denier de 4,4 cependant que les fibres de la seconde couche 3 de type cardé ont un denier de 1,7.

On peut utiliser pour les couches de fibres de type cardé des fibres textiles naturelles ou synthétiques, telles que des fibres de cellulose, de viscose, de polyester, de polyéthylène, de polypropylène, de nylon, et de copolymères éthylène-propylène. On peut également utiliser des fibres bi-composants, ou des fibres mélangées avec un liant tel qu'une colle réactivable à chaud ou une poudre de polymère à bas point de fusion. Les fibres bi-composants sont des fibres comprenant une âme en un polymère à point de fusion relativement élevé enveloppée par un autre polymère de point de fusion relativement bas permettant une fusion de la surface de la fibre avec un chauffage modéré, tel qu'un chauffage à l'air chaud. De telles fibres sont bien connues dans la technique. Un exemple de fibres bi-composants est une fibre comprenant une âme de polyester enveloppée de polyoléfine, par exemple de polyéthylène.

Les colles réactivables à chaud sont bien connues dans la technique, un exemple d'une telle colle est une solution aqueuse de type EVA ou acrylique.

De même les poudres à bas point de fusion utilisables dans la présente invention sont bien connues dans la technique et sont généralement des poudres de polymères à bas point de fusion, tel que, par exemple une poudre de polyéthylène.

Les couches de fibres de type cardé du matériau composite de l'invention peuvent être constituées de fibres de même nature ou de natures différentes, ayant des deniers identiques ou différents, à la condition que le denier des fibres de la première couche 2 soit supérieur au denier des fibres de la seconde couche 3.

En particulier, et de préférence, chacune des couches est constituée par un mélange de fibres peu ou pas fusible, tel que des fibres de polyester, avec des fibres à bas point de fusion telles que des fibres de polyoléfine, par exemple de polyéthylène, ou des fibres bi-composants, ou encore de fibres mélangées avec un liant tel qu'une colle réactivable à chaud ou poudre à bas point de fusion. De préférence, la proportion de fibres à bas point de fusion ou à bi-composants ou encore la quantité de liant mélangé aux fibres sera comprise entre 5 et 30% en poids par rapport au poids total.

Chacune des couches peut également être constituée par des fibres de nature identique.

Egalement, la nature des fibres peut être identique ou différente d'une couche à l'autre.

Bien évidement, si les deux couches sont constituées uniquement de fibres peu ou pas fusibles, telles que des fibres de polyester, les couches, dans le produit final sont réunies uniquement par aiguilletage.

Les couches de fibres de type cardé 2, 3 peuvent avoir un grammage identique ou différent. De préférence les couches ont un grammage compris entre 10 et 30 g/m².

Les couches de fibres de type cardé 2 et 3 sont réunies ensembles par aiguilletage. De préférence, l'aiguilletage a une densité comprise entre 10 et 100 coups d'aiguille /cm² et est obtenu à partir des deux faces du matériau composite. Cet aiguilletage non seulement assure une bonne liaison entre les deux couches du matériau composite mais également permet d'acroître l'orientation verticale des fibres, favorisant ainsi le temps de transpercement du composite par les liquides corporels.

Lorsque au moins une des couches de fibres de type cardé se compose de fibres à bas point de fusion, telles que des fibres de polyoléfine, par exemple de polyéthylène, de fibres bi-composants, ou de fibres mélangées avec un liant, ou d'un mélange comportant de telles fibres, la liaison entre les couches de fibres de type cardé peut également s'effectuer par thermofusion, en complément de l'aiguilletage précédent.

De manière recommandée on soumet les couches à un aiguilletage avant la réactivation à l'air chaud pour la thermofusion des fibres, car cet aiguilletage en augmentant le taux d'orientation des fibres selon la verticale améliore la réactivation par l'air chaud traversant les fibres et leur fixation entre-elles.

Dans une autre réalisation (non représentée) le matériau composite selon l'invention comprend, en plus des première et deuxième couches de fibres de type cardé, une troisième couche en non-tissé classique, perméable aux fluides, de faible grammage. Cette troisième couche en non-tissé, supplémentaire, peut être constituée par tout non-tissé de type classique, bien connu dans la technique, tel que par exemple un non-tissé de type lié au filage (spun) ou lié par fusion.

Le non-tissé classique peut être formé de fibres textiles naturelles ou synthétiques, telles que des fibres de cellulose, de viscose, de polyester, de polyéthylène, de polypropylène, de nylon, ou de copolymères éthylène-propylène.

De préférence, le non-tissé de type classique est constitué par une couche de fibres, enduite sur une de ses faces avec une colle réactivable à chaud ou est constitué d'un mélange de fibres comprenant des fibres choisies parmi les fibres à bas point de fusion, les fibres mélangées à un liant tel qu'une colle réactivable à chaud ou une poudre à bas point de fusion. Lorsque le non-tissé classique est un mélange, la proportion de fibres à bas point de fusion ou de fibres mélangées à un liant est comprise entre 5 et 30% en poids par rapport au poids total. Comme mentionné précédemment, ce non-tissé classique est de faible grammage, généralement compris entre 10 et 20 g/m², et de préférence de 15 g/m².

Le non-tissé de type classique est lié par thermofusion à la seconde couche de fibres de type cardé de plus faible denier, par exemple par passage des couches du composite dans une calandre de contre-collage du type STORCK.

Un procédé de fabrication du matériau non-tissé composite selon l'invention consiste à alimenter une carde dans le sens de la largeur avec des premières fibre et des secondes fibres, les premières fibres ayant un denier supérieur aux secondes fibres, pour former des bandes adjacentes ou juxtaposées, puis à partir de cette carde à alimenter avec les bandes adjacentes une machine à croiser les nappes de carde appelée étaleur-nappeur, par exemple une machine type 350 de la société ASSELIN, qui a pour objet de superposer les bandes. Les bandes superposées sont ensuite fournies à un dispositif étireur de nappe, par exemple, une étireuse de nappe de la société ASSELIN qui étire les fibres pour les orienter préférentiellement dans le sens de défilement de la machine et augmente l'orientation verticale des fibres, pour obtenir une nappe formée d'une première couche des premières fibres et d'une deuxième couche des deuxièmes fibres. La nappe est ensuite soumise à un aiguilletage seul ou à un aiguilletage et à une réactivation par air chaud à travers les couches de fibres pour lier les fibres entre-elles.

Le procédé de l'invention peut en outre comporter une étape supplémentaire dans laquelle, la nappe constituée des deux couches de fibres de type cardé est liée à une troisième couche d'un non-tissé classique, de faible grammage, par thermofusion de cette troisième couche de non-tissé classique avec la seconde couche de fibres de type cardé de la nappe, c'est-à-dire la couche formée des fibres de plus faible dernier. De préférence, cette thermofusion s'effectue par passage de la nappe de fibres de type cardé et de la troisième couche en non-tissé classique dans une calandre de contre-collage, par exemple du type STORCK.

L'orientation des fibres dans le matériau composite de type cardé de l'invention, favorise le transport des liquides corporels dans le sens de la longueur et dans le cas de la fabrication de couche-culottes avec un voile de surface ou une bande de zone d'entre-jambes placé dans le sens de la longueur, fabriqué à partir de ce matériau non-tissé composite, permet d'utiliser avantageusement une plus grande proportion de la masse absorbante du coussin placé en dessous.

Cet étirage de la nappe de fibres de type cardé est suivi par un aiguilletage qui a encore pour effet d'accroître le taux d'orientation verticale des fibres et qui améliore le temps de traversée du composite par les liquides, tout en assurant une bonne liaison entre les couches.

Lorsque le non-tissé composite selon l'invention comporte en outre une troisième couche en non-tissé classique, on accroît encore la résistance au peluchage du composite.

### Exemple de réalisation d'un matériau non-tissé composite de type cardé selon l'invention.

On alimente une carde dans le sens de la largeur avec des fibres comprenant principalement des fibres de polyester de 4,4 deniers (0,49 tex) et des fibres de polyester de 1,7 deniers (0,19 tex). Les fibres sont de fait soit constituées par un mélange de fibres de polyester et de fibres bi-composants comportant une âme de polyester et une enveloppe de polyéthylène, la proportion de fibres bi-composants étant de 5 à 30% en poids par rapport au poids total ou par un mélange de fibres de polyester et de polyéthylène de 4,4 (0,49 tex) et 1,7 deniers (0,19 tex), la proportion de fibres de polyéthylène étant de 5 à 30% en poids par rapport au poids total.

On alimente avec les bandes juxtaposées issues de la carde un dispositif étaleur-nappeur de type 350 de la Société ASSELIN pour obtenir une nappe de fibres de type cardé formée de bandes superposées de fibres de 1,7 deniers (0,19 tex) et de fibres de 4,4 deniers (0,49 tex). On alimenté avec ces bandes superposées une étireuse de nappe de la société ASSELIN pour obtenir une nappe composite formée par une première couche de fibres de 4,4 deniers (0,49 tex) et une seconde couche de fibres de 1,7 deniers (0,19 tex). On soumet la nappe de fibres à un aiguilletage ayant une densité d'aiguilletage de 20 coups /cm² puis on fait traverser la nappe de fibres de type cardé par un flux d'air chaud afin de réactiver les fibres bi-composants. On obtient un matériau non-tissé composite de type cardé selon la présente invention. Ce matériau présente un excellent temps de traversée par les liquides, une bonne résistance au remouillage et ne peluche pas.

D'autre part, lorsqu'il est utilisé comme voile de surface ou en complément d'un voile de surface, comme bande de zone d'entre-jambes, en plaçant la couche de fibres de 1,7 deniers (0,19 tex) vers l'intérieur, c'est-à-dire en contact avec la peau de l'utilisateur ou de la surface externe du voile de surface, et la couche de fibres de 4.4 deniers (0,49 tex) directement sur le coussin absorbant on obtient un article absorbant selon l'invention ayant une douceur convenable due à la finesse des fibres utilisées pour la couche interne.

Le matériau non-tissé composite obtenu selon l'invention présente également des caractéristiques mécaniques suffisantes pour être enroulé et utilisé sur une machine de transformation.

On a représenté figures 2 et 3, à titre d'exemple, un article d'hygiène absorbant, tel qu'une couche-culotte, comportant une bande d'entre-jambes constituée d'un matériau non-tissé de type cardé selon l'invention.

La couche-culotte représentée sur les figures 2 et 3 comprend de façon connue en soi, une feuille de support 10 imperméable aux liquides, un coussin absorbant 12, par exemple en pâte fluff de cellulose, éventuellement avec incorporation de matières polymères dites superabsorbantes, une bande de zone d'entre-jambes 27, une bande intermédiaire 29 de dimension analogue à celle de la bande de zone d'entre-jambes 27, disposée directement sur cette bande de zone d'entre-jambes, et généralement constituée d'un non-tissé classique, et une feuille de surface 13, par exemple un voile de non-tissé hydrophobe. Les deux feuilles 10 et 13 ont les mêmes dimensions et la même forme en sablier c'est-à-dire une forme rectangulaire avec deux échancrures latérales opposées délimitant, dans le sens de la longueur de la couche-culotte une zone 15 d'entre-jambes de largeur réduite entre deux zones d'extrémité 16 et 17 de largeur accrue. Le coussin absorbant 12 disposé entre les deux feuilles 10 et 13 présente également une forme en sablier mais de dimension plus faible que les feuilles 10 et 13 qui sont reliées entre-elles par exemple par collage sur tout le pourtour du coussin 12.

Des premiers éléments élastiques 18 longitudinaux, constitués par exemple chacun par un ou plusieurs brins ou fils élastiques ou par une bandelette élastique, sont fixés à l'état tendu à la feuille de support 10, au moins dans la zone d'entre-jambes 15 entre le fond des échancrures de la feuille 10 et le fond des échancrures correspondantes du coussin absorbant 12.

Par ailleurs, des attaches adhésives 19 sont fixées sur les bords latéraux opposés sur la zone d'extrémité 16 constituant la partie arrière de la couche-culotte.

La feuille de surface 13 comporte à sa partie médiane une découpe longitudinale médiane 25 pour former une ouverture, de préférence de forme oblongue. La largeur de l'ouverture est nettement inférieure à la largeur du coussin 12, de préférence inférieure à la moitié de la largeur du coussin 12 dans la zone d'entre-jambes. De plus, la feuille de surface 13 comporte, sur toute sa longueur, deux doubles plis en Z 30 vers le bas (vers le coussin 12) disposés de part et d'autre de l'ouverture 25, et des éléments élastiques 26 sont fixés à l'état tendu par collage dans les replis supérieurs de ces doubles plis 30, les deux replis de chaque double pli 30 étant solidarisés l'un à l'autre par collage ou tout autre moyen de façon à consolider ou rigidifier la feuille de couverture 13 à cet endroit et faciliter ainsi la découpe avec enlèvement de matière de l'ouverture 25, pratiquée ultérieure-ment. La bande d'entre-jambes 27 en matériau non-tissé composite selon l'invention est disposée directement au-dessus du coussin absorbant sur toute la longueur de la couche-culotte et a une largeur au moins égale à la largeur du coussin absorbant 12 dans la zone d'entre-jambes. La bande de zone d'entre-jambes 27 est liée au coussin absorbant 12 et/ou à la bande intermédiaire 29 par tout moyen approprié bien connu, et la bande intermédiaire 29, dans la réalisation représentée, est fixée à la feuille de surface 13, d'une part sur les deux bords transversaux de la couche-culotte et d'autre part suivant deux lignes longitudinales 28, par exemple des lignes de collage, situées dans une position légèrement décalée par rapport aux bords longitudinaux du coussin absorbant 12 dans la zone d'entre-jambes 15.

Bien qu'on ait représenté la feuille de surface comme comportant deux doubles plis en Z 30, il est également possible de modifier la forme de ces plis de toute manière appropriée, par exemple en réalisant des plis ayant la forme d'un S.

Egalement, les éléments élastiques 26 peuvent être disposés en tout endroit approprié autre que les replis supérieurs, par exemple les replis inférieurs.

La bande d'entre-jambes 27 est constituée par un matériau non-tissé composite selon l'invention comprenant deux couches de fibres de type cardé de deniers différents réunies par aiguilletage, la couche de fibres de type cardé de denier le plus élevé étant en contact direct avec le coussin absorbant 12.

Cette couche-culotte présente une vitesse élevée de traversée par les liquides, une bonne résistance au remouillage et ne peluche pas.

## Revendications

1. Matériau non-tissé présentant une vitesse de traversée par les liquides et une résistance au remouillage améliorées composite caractérisé en ce qu'il comprend une nappe de fibres de type cardé perméable aux fluides corporels, comportant au moins une première couche de fibres de type cardé et une seconde couche de fibres de type cardé, les fibres de la première couche ayant un denier supérieur aux fibres de la seconde couche, les couches étant réunies entre elles par aiguilletage.

2. Matériau selon la revendication 1, caractérisé en ce que les fibres de la première couche ont un denier compris entre 3 et 8 (tex compris 0,33 et 0,89) et les fibres de la seconde couche ont un denier compris entre 1 et 3 (0,11 tex et 0,33 tex).

3. Matériau selon la revendication 1 ou 2, caractérisé en ce que les fibres de la première couche sont choisies parmi les fibres de cellulose, de viscose, de polyester, de polyéthylène, de polypropylène, de nylon, les copolymères d'éthylène-propylène, les fibres bi-composants, et les fibres mélangées avec un liant tel qu'une colle réactivable à chaud ou une poudre à bas point de fusion.

4. Matériau selon la revendication 1 ou 2, caractérisé en ce que les couches sont constituées de fibres de polyester.

5. Matériau selon la revendication 1 ou 2, caractérisé en ce que les couches sont constituées de fibres de polyester mélangées à un liant tel qu'une colle réactivable à chaud ou une poudre à bas point de fusion et sont en outre liées par thermofusion.

6. Matériau selon la revendication 1 ou 2, caractérisé en ce qu'au au moins une des couches comprend des fibres bi-composants, des fibres mélangées à un liant tel qu'une colle réactivable à chaud ou une poudre à bas point de fusion ou des fibres à bas point de fusion et les couches sont en outre liées par thermofusion.

7. Matériau selon la revendication 6, caractérisé en ce que les deux couches comprennent des fibres bi-composants, des fibres mélangées à un liant tel qu'une colle réactivable à chaud ou une poudre à bas point de fusion ou des fibres à bas point de fusion.

8. Matériau selon la revendication 1 ou 2, caractérisé en ce que les couches comprennent des mélanges de fibres de polyester et de fibres choisies parmi les fibres bi-composants ou à bas point de fusion et sont en outre liées par thermofusion.

9. Matériau non-tissé composite selon l'une quelconque des revendications 1 à 8 caractérisé en ce qu'il comprend en outre une troisième couche d'un non-tissé classique, de faible grammage, lié par thermofusion à l'une des couches de fibres de type cardé.

10. Matériau composite selon la revendication 9 caractérisé par le fait que la troisième couche de non-tissé classique est liée à ladite couche de fibres de type cardé par une face enduite d'une colle réactivable à chaud.

11. Procédé de fabrication d'un matériau non-tissé composite, caractérisé en ce qu'il comprend les étapes consistant à:
- alimenter une carde, dans le sens de la largeur, avec des premières fibres et des secondes fibres, les premières fibres ayant un denier supérieur aux secondes fibres, pour former des bandes adjacentes de fibres de type cardé des premières et secondes fibres;
- fournir les bandes adjacentes de fibres de type cardé à un dispositif étaleur-nappeur pour croiser les bandes et former une nappe constituée des bandes superposées des premières et secondes fibres;
- fournir cette nappe à un dispositif étireur de nappe pour étirer les fibres des bandes et les orienter dans le sens du défilement tout en augmentant le taux d'orientation verticale desdites fibres afin d'obtenir à la sortie du dispositif étireur de nappe une nappe de fibres comprenant une première couche des premières fibres et une seconde couche des secondes fibres ; et
- aiguilleter la nappe de fibres obtenue depuis au moins l'une de ses faces.

12. Procédé selon la revendication 11, caractérisé en ce que la densité d'aiguilletage est comprise entre 10 et 100 coups d'aiguille/cm².

13. Procédé selon la revendication 11 ou 12, caractérisé en ce que au moins une des couches de la nappe comprend des fibres choisies parmi les fibres bi-composants, les fibres mélangées à un liant tel qu'une colle réactivable à chaud ou une poudre à bas point de fusion et les fibres à bas point de fusion et en ce qu'il comprend en outre une réactivation par air chaud traversant la nappe pour lier les fibres des couches entre elles.

14. Procédé selon l'une quelconque des revendications 11 à 13 caractérisé en ce qu'il comprend en outre une étape consistant à lier par thermofusion une troisième couche d'un non-tissé classique à une des couches de fibres de type cardé.

15. Procédé selon la revendication 14 caractérisé en ce que l'étape de liaison par thermofusion de la troisième couche de non-tissé classique à ladite couche de fibres de type cardé est réalisée au moyen d'une calandre de contre-collage.

16. Article d'hygiène absorbant, par exemple une couche-culotte, comprenant une couche externe imperméable aux liquides corporels (10), un coussin absorbant (12) perméable aux fluides corporels fixé à la couche externe, la couche externe (10) et le coussin absorbant (12) comportant des parties d'extrémité (16, 17) élargies réunies par une zone d'entre-jambes (15) de plus faible largeur, une feuille de surface (13), de préférence hydrophobe, et une bande de zone d'entre-jambes perméable aux fluides corporels (27) disposée entre le coussin absorbant (12) et la feuille de surface (13) et de largeur analogue à la largeur du coussin dans la zone d'entre-jambes et de longueur au moins égale à la longueur du coussin et liée au coussin, caractérisé en ce que la bande de zone d'entre-jambes est constituée du matériau composite selon l'une quelconque des revendications 1 à 10, la première couche de fibres de plus grand denier étant directement en contact avec le coussin absorbant, lorsque le matériau composite est constitué uniquement de couches de fibres de type cardé.

17. Article d'hygiène absorbant selon la revendication 16 dans lequel la feuille de surface (13) comporte une découpe longitudinale médiane (25) formant une ouverture, de préférence de forme oblongue, et l'article comportant en outre une bande intermédiaire (29) en non-tissé classique, de dimension analogue à la bande de zone d'entre-jambes (27), et disposée directement sur la bande de zone d'entre-jambes (27).

## Patentansprüche

1. Verbund aus Vliesstoff-Material mit verbesserter Durchgangsgeschwindigkeit für Flüssigkeiten und mit verbesserter Durchfeuchtungbesändigkeit, welcher dadurch gekennzeichnet ist, daß er ein für Körperflüssigkeiten durchlässiges Vlies aus Fasern vom kardierten Typ umfaßt, welches mindestens eine erste Schicht aus Fasern vom kardierten Typ und eine zweite Schicht aus Fasern vom kardierten Typ aufweist, wobei die Fasern der ersten Schicht einen höheren Denier-Wert als die Fasern der zweiten Schicht aufweisen, und worin die Schichten untereinander durch Nadelung verbunden sind.

2. Material gemäß Anspruch 1,
dadurch **gekennzeichnet,** daß
die Fasern der ersten Schicht einen Denier-Wert von 3 bis 8 (0,33 bis 0,89 tex) und die Fasern der zweiten Schicht einen Denier-Wert von 1 bis 3 (0,11 bis 0,33 tex) aufweisen.

3. Material gemäß Anspruch 1 oder 2,
dadurch **gekennzeichnet**, daß
die Fasern der ersten Schicht aus Fasern aus Cellulose, Viskose, Polyester, Polyethylen, Polypropylen, Nylon, Copolymeren von Ethylen-Propylen, Bikomponentenfasern und aus Fasern ausgewählt sind, die mit einem Bindemittel wie einem wärmeaktivierbaren Klebstoff oder einem Pulver mit niedrigem Schmelzpunkt vermischt sind.

4. Material gemäß Anspruch 1 oder 2,
dadurch **gekennzeichnet**, daß
die Schichten aus Polyesterfasern zusammengesetzt sind.

5. Material gemäß Anspruch 1 oder 2,
dadurch **gekennzeichnet**, daß
die Schichten aus Polyesterfasern, die mit einem Bindemittel wie einem wärmeaktiverbaren Klebstoff oder einem Pulver mit niedrigem Schmelzpnkt vermischt sind, zusammengesetzt und ausserdem durch Thermofusion verbunden sind.

6. Material gemäß Anspruch 1 oder 2,
dadurch **gekennzeichnet** daß
mindestens eine der Schichten Bikomponentenfasern, Fasern, die mit einem Bindemittel wie einem wärmeaktivierbaren Klebstoff oder einem Pulver mit niedrigem Schmelzpunkt vermischt sind, oder Fasern mit niedrigem Schmelzpunkt enthält, und daß die Schichten ausserdem durch Thermofusion verbunden sind.

7. Material gemäß Anspruch 6,
dadurch **gekennzeichnet**, daß
die beiden Schichten Bikomponentenfasern, Fasern, die mit einem Bindemittel wie einem wärmeaktivierbaren Klebstoff oder einem Pulver mit niedrigem Schmelzpunkt vermischt sind, oder Fasern mit niedrigem Schmelzpunkt enthalten.

8. Material gemäß Anspruch 1 oder 2,
dadurch **gekennzeichnet**, daß
die Schichten Mischungen aus Polyesterfasern und Fasern, die aus Bikomponentenfasern oder aus Fasern mit niedrigem Schmelzpunkt ausgewählt sind, enthalten und ausserdem durch Thermofusion verbunden sind.

9. Vliesstoff-Verubndmaterial gemäß jedem der Ansprüche 1 bis 8,
dadurch **gekennzeichnet**, daß
es ausserdem eine dritte Schicht aus klassischem Vliesstoff mit geringem Grammgewicht umfaßt, welche durch Thermofusion mit einer der Schichten aus Fasern vom kardierten Typ verbunden ist.

10. Verbundmaterial gemäß Anspruch 9,
dadurch **gekennzeichnet,** daß
die dritte Schicht aus klassischem Vliesstoff mit der genannten Schicht aus Fasern vom kardierten Typ verbunden ist, die auf einer Seite mit einem wärmeaktivierbaren Klebstoff überzogen ist.

11. Verfahren zur Herstellung eines Vliesstoff-Verbundmaterials,
dadurch **gekennzeichnet**, daß
Stufen enthalten sind, in denen man:
- eine Karde (Streichmaschine), in Richtung der Ausbreitung, mit ersten Fasern und zweiten Fasern, deren erste Fasern einen höheren Denier-Wert als die zweiten Fasern aufweisen, zur Bildung aneinander liegender Bahnen aus Fasern vom kardierten Typ aus den ersten und zweiten Fasern beschickt;
- man die aneinander liegenden Bahnen aus Fasern vom kardierten Typ einer Vlies-Ausbreitvorrichtung zuführt, um die Bahnen zu köpern und ein Vlies aus übereinander angeordneten Bahnen der ersten und zweiten Fasern zu bilden;
- man dieses Vlies einer Vlies-Ziehvorrichtung zuführt, um die Fasern der Bahnen zu recken und sie in Richtung der Ausbreitung zu orientieren, wobeiman insgesamt dabei den vertikalen Orientierungsgehalt der genannten Fasern erhöht, um schließlich beim Austritt aus der Vliesbahn-Ziehvorrichtung ein Faservlies zu erhalten, das eine erste Schicht aus ersten Fasern und eine zweite Schicht aus zweiten Fasern umfaßt; und
- man das erhaltene Faservlies mindestens von einer seiner Seiten her nadelt.

12. Verfahren gemäß Anspruch 11,
dadurch **gekennzeichnet**, daß
die Nadelungsdichte 10 bis 100 Nadelstiche/cm² ausmacht.

13. Verfahren gemäß Anspruch 11 oder 12,
dadurch **gekennzeichnet**, daß
mindestens eine der Vliesschichten Fasern enthält, die aus Bikomponentenfasern, aus Fasern, die mit einem Bindemittel wie einem wärmeaktivierbaren Klebstoff oder einem Pulver mit niedrigem Schmezpunkt vermischt sind, und aus Fasern mit einem niedrigen Schmelzpunkt ausgewählt sind, und daß ausserdem eine Aktivierung mit Warmluft durchgeführt wird, welche das Vlies durchströmt, um die Fasern der Schichten untereinander zu verbinden.

14. Verfahren gemäß einem der Ansprüche 11 bis 13,
dadurch **gekennzeichnet**, daß
es ausserdem eine Stufe aufweist, in der man durch Thermofusion eine dritte Schicht aus einem klassischen Vliesstoff mit einer der Schichten aus Fasern vom kardierten Typ verbindet.

15. Verfahren gemäß Anspruch 14,
dadurch **gekennzeichnet**, daß
die Stufe zur durch Thermofusion erfolgenden Verbindung der dritten Schicht aus klassischem Vliesstoff mit der genannten Schicht aus Fasern vom kardierten Typ mit einem unter Gegenverklebung betriebenen Kalander durchgeführt wird.

16. Absorbierender Hygieneartikel, wie z.B. ein Windel-Höschen, umfassend eine für Körperflüssigkeiten undurchlässige Aussenschicht (10), ein an die Aussenschicht fixiertes, für Körperflüssigkeiten durchlässiges absorbierendes Kissen (12), deren Aussenschicht (10) und absorbierendes Kissen (12) verbreiterte Aussenteilstücke (16, 17) aufweisen, die über eine Zwischenbeinzone (15) geringerer Breite miteinander in Verbindung stehen, ferner umfassend eine Oberflächenfolie (13), die vorzugsweise hydrophob ist, sowie eine für Körperflüssigkeiten durchlässige, für die Zone zwischen den Beinen vorgesehene Bahn und/oder Binde (27), die zwischen dem absorbierenden Kissen (12) und der Oberflächenfolie (13) angeordnet sind und die gleiche Breite wie die Breite des Kissens in der Zwischenbeinzone sowie die mindestens gleiche Länge wie die Länge des Kissens aufweisen und mit dem Kissen verbunden sind,
dadurch **gekennzeichnet,** daß
die Binde für die Zwischenbeinzone aus dem Verbundmaterial gemäß jedem der Ansprüche 1 bis 10 zusammengesetzt ist, wobei die erste Schicht aus Faserne ines größeren Denier-Werts direkt in Kontakt mit dem absorbierenden Kissen vorliegt, wenn das Verbundmaterial nur aus Schichten aus Fasern vom kardierten Typ zusammengesetzt ist.

17. Absorbierender Hygieneartikel gemäß Anspruch 16, worin die Oberflächenfolie (13) einen mittleren Längsausschnitt (25) aufweist, der eine Öffnung, vorzugsweise länglicher Form, bildet, wobei der Artikel ausserdem eine Zwischenbahnbinde (29) aus klassischem Vliesstoff in gleicher Abmessung wie die für die Zwischenbeinzone vorgesehene Binde (27) aufweist, die direkt über der Bindenbahn (27) für die Zwischenbeinzone angeordnet ist.

## Claims

1. Composite nonwoven material through which liquid can pass at a high speed and having an excellent resistance to rewetting and fluffing ,characterized in that it comprises a sheet of fibers of carded type which is permeable to body fluids , comprising at least one first layer of fibers of carded type and a second layer of fibers of carded type, the fibers of the first layer having a higher denier than the fibers of the second layer, the layers being joined together by needling.

2. Material according to claim 1, characterized in that the fibers of the first layer have a denier of between 3 and 8 (0.33-0.89 tex) and the fibers of the second layer have a denier of between 1 and 3 (0.11-0.39 tex).

3. Material according to claim 1 or 2, characterized in that the fibers of the first layer are chosen from cellulose, viscose, polyester, polyethylene, polypropylene, nylon, ethylene-propylene copolymer fibers, two-component fibers and fibers mixed with a binder such as a heat-reactivable adhesive or a powder of low melting point.

4. Material according to claim 1 or 2, characterized in that the layers consist of polyester fibers.

5. Material according to claim 1 or 2, characterized in that the layers consist of polyester fibers mixed with a binder such as a heat-reactivable adhesive or a powder of low melting point and are additionally bonded by heat-melting.

6. Material according to claim 1 or 2, characterized in that at least one of the layers comprises two-component fibers, fibers mixed with a binder such as a heat-reactivable adhesive or a powder of low melting point or fibers with a low melting point and the layers are additionally bonded by heat-melting.

7. Material according to claim 6, characterized in that the two layers comprise two-component fibers, fibers mixed with a binder such as a heat-reactivable adhesive or a powder of low melting point or fibers with a low melting point.

8. Material according to claim 1 or 2, characterized in that the layers comprise mixtures of polyester fibers and of fibers chosen from two-component fibers or with a low melting point and are additionally bonded by heat-melting.

9. Composite nonwoven material according to any one of claims 1 to 8, characterized in that it additionally comprises a third layer of a conventional nonwoven, of low weight per unit area, bonded by heat-melting to one of the layers of fibers of carded type.

10. Composite material according to claim 9, characterized in that the third layer of conventional nonwoven is bonded to said layer of fibers of carded type by a side coated with a heat-reactivable adhesive.

11. Process for the manufacture of a composite nonwoven material, characterized in that it comprises the stages consisting in:
- feeding a card, in the width direction, with first fibers and second fibers, the first fibers having a higher count than the second fibers, to form adjacent strips of fibers of carded type from the first and second fibers;
- supplying the adjacent strips of fibers of carded type to a spreader-lapper device to cross the strips and to form a sheet consisting of superposed strips of the first and second fibers;
- supplying this sheet to a sheet-stretcher device to stretch the fibers of the strips and to orient them in the direction of travel while increasing the degree of vertical orientation of said fibers in order to obtain, at the exit of the sheet-stretcher device, a sheet of fibers comprising a first layer of the first fibers and a second layer of the second fibers; and
- needling the sheet of fibers which is obtained from at least one of its sides.

12. Process according to claim 11, characterized in that the density of needling is between 10 and 100 needle strokes/cm².

13. Process according to claim 11 or 12, characterized in that at least one of the layers of the sheet comprises fibers chosen from two-component fibers, fibers mixed with a binder such as a heat-reactivable adhesive or a powder of low melting point and fibers with a low melting point and in that it additionally comprises a reactivation by hot air passing through the sheet to bond the fibers of the layers to each other.

14. Process according to any one of claims 11 to 13, characterized in that it additionally comprises a stage consisting in bonding a third layer of a conventional nonwoven by heat-melting to one of the layers of fibers of carded type.

15. Process according to Claim 14, characterized in that the stage of bonding of the third layer of conventional nonwoven by heat-melting to said layer of fibers of carded type is carried out by means of an adhesive-backing calender.

16. Absorbent article of hygiene, for example a diaper, comprising an outer layer which is impervious to body liquids (10), an absorbent pad (12) which is permeable to body fluids and attached to the outer layer, the outer layer (10) and the absorbent pad (12) comprising widened end parts (16, 17) joined by a crotch region (15) of smaller width, a preferably hydrophobic surface sheet (13) and a crotch region strip which is permeable to body fluids (27) and arranged between the absorbent pad (12) and the surface sheet (13) and of width similar to the width of the pad in the crotch region and of length which is at least equal to the length of the pad and bonded to the pad, characterized in that the crotch region strip consists of the composite material according to any one of claims 1 to 10, the first layer of fibers of higher count being directly in contact with the absorbent pad when 'the composite material consists solely of layers of fibers of carded type.

17. Absorbent article of hygiene according to claim 16, in which the surface sheet (13) comprises a median lengthwise cutout (25) forming an opening, preferably of oblong shape, and the article additionally comprising an intermediate strip (29) of conventional nonwoven, similar in size to the crotch region strip (27) and arranged directly on the crotch region strip (27).
